# EUROPEAN PATENT SPECIFICATION

(11) **EP 1 773 431 B1**
(45) Date of publication and mention of the grant of the patent: **18.02.2009**
(21) Application number: 05775757.7
(22) Date of filing: 22.07.2005
(51) Int. Cl.: A61M 5/31, A61J 1/00, A61M 5/28

(54) **MEDICAL RECONSTITUTION SYSTEM**
MEDIZINISCHES REKONSTITUTIONSSYSTEM
SYSTEME DE RECONSTITUTION MEDICALE

(30) Priority: 24.07.2004 DE 102004036051; 02.09.2004 US 606761 P
(43) Date of publication of application: 18.04.2007
(73) Proprietor: Bayer HealthCare, LLC, Tarrytown, NY 10591 (US)
(72) Inventor: MUELLER-BECKHAUS, Andreas, Walnut Creek, California 94598 (US); MONTELLATO, Randy, San Rafael, California 94901 (US); VANTRIESTE, Martin, Blackhawk, California 94506 (US)
(74) Representative: Linhart, Angela
(86) International application number: PCT/US2005/026298
(87) International publication number: WO 2006/012611

(56) References cited:
- EP-A- 0 242 956
- WO-A-20/04041148
- GB-A- 190 924 783
- US-A- 3 729 031
- US-A- 3 786 811
- US-A- 3 885 297
- US-A- 5 364 369
- US-A- 5 697 918
- US-A- 5 772 665
- US-A- 6 070 623

## Description

### FIELD OF THE INVENTION

The invention relates to medical devices and systems, and more particularly relates to an injection syringe that is particularly suited for use with a receptacle of the type having a self-contained movable piercing member.

### BACKGROUND

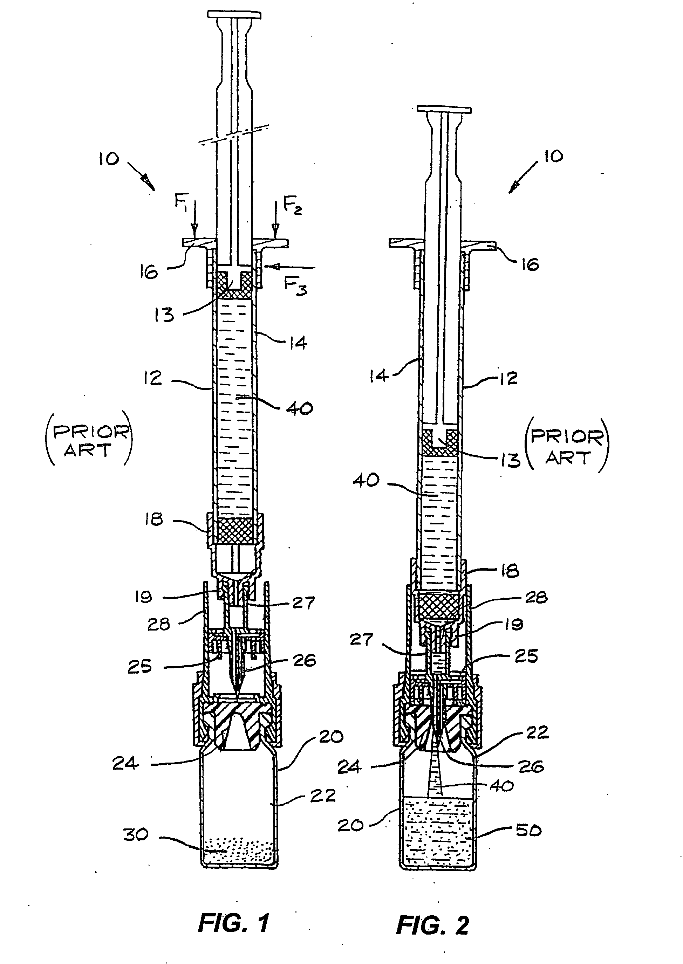

Medical reconstitution devices and systems are known for safely and effectively delivering two-part, reconstituted pharmaceutical compositions for injection into a patient. One embodiment 10 of such a device and system is shown in Figures 1 and 2, and is described in detail in U.S. Patent No. 6,070,623, issued June 6, 2000, and assigned to Biodome America, Inc. (Princeton, N.J.). It is marketed under the mark BIO-SET®. As shown in Figure 1, such a prior art system 10 includes an injection syringe 12 and a receptacle 20. The receptacle 20 includes a vial 22 containing a first constituent 30 in powder or particulate form, for example. The syringe 12 includes a barrel 14 containing a second constituent 40 in a flowable form, such as a liquid diluent, for example. The system 10 is used to mix and recombine the first and second constituents 30, 40 to form a desired pharmaceutical composition 50 for subsequent injection into a patient using the syringe 12.

As shown in Figures 1 and 2, the receptacle 20 includes a vial 22 that is closed and sealed by a piercable seal plug or stopper 24 to contain and preserve the first constituent 30 in the vial 22. The seal plug 24 may be formed of a piercable synthetic or rubber material, for example. As shown in Figure 1, a movable piercing member 25 is positioned above and aligned with the seal plug 24, and includes a centrally positioned piercing member needle 26. The needle 26 is in fluid communication with a first luer-type connector 27 positioned at the upper end of the movable piercing member 25. A sterile shield 28 substantially surrounds the movable piercing member 25, needle, 26, and first connector 27. In Figure 1, the movable piercing member 25 and needle 26 are shown in a fully-retracted position.

As shown in Figures 1 and 2, the syringe 12 has a conventional construction including a barrel 14, a proximal finger brace 16, a distal end member 18, and a plunger 13. The distal end member 18 includes a second connector 19 that is in selective fluid communication with the interior of the barrel 14. The second connector 19 is a luer-type connector configured for mating, sealing engagement with the first connector 27 on the receptacle 20. The second connector 19 also is configured for connection to other compatible medical devices such as an intravenous tube fitting or an injection needle, for example. The syringe barrel 14 typically is constructed of glass, and the proximal finger brace 16 and distal end member 18 typically are constructed of a suitable polymeric material. The syringe 12 is pre-filled with a second flowable constituent 40, such as a diluent, for example.

In use, the second connector 19 on the syringe 12 and the first connector 27 on the receptacle 20 are aligned and sealingly engaged together as shown in Figure 1. An axial load is applied in a distal direction to the proximal finger brace 16 of the syringe 12 as indicated by arrows F₁ and F₂ in Figure 1, thereby urging the syringe 12 and movable piercing member 25 in a distal direction toward the seal plug 24 in the receptacle 20. The axial load applied to the proximal finger brace 16 causes the syringe 12 and movable piercing member 25 to move together until the piercing member needle 26 pierces and penetrates the seal plug 24 as shown in Figure 2. Once the movable piercing member 25 is in a fully-pierced position as shown in Figure 2, a fluid communication path is established between the vial 22 and syringe barrel 14 through the piercing member needle 26 and the engaged connectors 19, 27. In this fully-pierced position, the flowable second constituent 40 in the syringe 12 can be injected into the vial 22 by advancing the plunger 13 in a distal direction. The flowable second constituent 40 from the syringe 12 mixes with the first constituent 30 in the vial 22 to form a desired reconstituted composition 50. Once thoroughly mixed, the reconstituted composition 50 is drawn into the syringe by drawing the plunger 13 in a proximal direction. The syringe 12 is then ready for use with a compatible injection needle (not shown) to inject the reconstituted medical composition 50 into a patient.

Though a medical reconstitution system 10 like that described above provides a convenient and effective system for reconstituting first and second constituent ingredients into a medical composition for injection into a patient, such a system 10 has at least one shortcoming. In some cases, a transverse or eccentric load (such as that indicated by arrow F₃ in Figure 1) may inadvertently be applied at or near the proximal end of the syringe 12 as the syringe 12 is used to push the movable piercing member 25 toward the receptacle 20. Due to the length of the syringe 12, such an eccentric load F₃ may create a substantial bending moment at the distal end of the syringe at the engaged connectors 19, 27. Such an eccentric load "F₃" also may create a substantial bending moment in the syringe barrel 14. If the resultant bending loads are sufficiently large, at least a portion of the system 10 may be distorted, damaged, or broken. In a severe case, the syringe tip 19 or glass barrel 14 may break under the resultant bending stresses, thereby subjecting a user to a potentially severe safety hazard.

US 3,786,811 discloses a compact syringe construction wherein the internal plunger is externally actuated by a side-mount actuator so connected to an interior plunger portion that a forward advance of the actuator will effect a forward advance of the interior plunger, this for desired liquid expression from the syringe. Finger tabs are disclosed which extend from two opposite sides from the barrel of the syringe close to its injection end. The finger tabs are configured to permit application of a force by one or more fingers of a persons hand such that a move of the whole syringe is prevented during injection e.g. application of a force to the plunger in the direction of injection.

GB 24,783 discloses a dental syringe fitted with a finger grip bar which is adjustable to various positions on the syringe barrel.

US 5,772,665 discloses a device for mixing a pharmaceutical composition stored in a chamber in said device with an agent to be added shortly before administration to form a preparation to be administered. The device comprises a hollow body having an outlet sealed by a removable closure and a plunger, which is slidable therein in sealing contact with the inner wall of the hollow body. The plunger, the hollow body and the removable closure define said chamber. An actuating means is connected to the plunger for displacing the same in the hollow body. A filling conduit for said agent is connected to the chamber. A check valve is associated with the conduit and the chamber, which prevents flow from the chamber but permits flow into said chamber through said conduit.

US 5,364,369 discloses a prefilled syringe for one or two component medicaments based upon the use of a vial containing a medicament or one component of a medicament, the vial having an open bottom closed by a piston. When a flexible extension of the piston is coupled with a tubular plunger, and an adaptor cap having an internal needle and an external connection for a needle is placed over a cap of the vial, the latter is converted into a prefilled syringe. The piston may have an axial passage closed by a resealable septum, so that a separate diluent stored in a flexible capsule may be introduced into the vial through the piston by a double ended needle mounted on a further cap applied to the capsule, the further cap being coupled within the tubular interior of the plunger so that the double ended needle penetrates the septum in the piston. The capsule is pushed forward onto the double ended needle when its contents are to be expelled into the vial. The capsule and its cap are then removed and discarded. The rear ends of the cap and a sleeve are formed with radially extending flanges respectively which form finger grips for operation of the syringe. Thus if a user grips the syringe by the flanges and presses them towards each other, the contents of the syringe can be expelled through the needle.

WO 2004/041148 Al discloses an assembly for transferring a liquid between a vial and a syringe. The assembly includes a housing having a central portion, the housing being open at one end and having a vial socket at the other opposite end adapted to receive and retain a vial having a penetrable closure. The housing further includes a sleeve located within the central portion of the housing. The sleeve has a first portion, a second portion adjacent the first portion, and a shoulder between the first portion and the second portion. The assembly also includes a protractible luer adaptor with a central hub having a first axial end and a second opposite axial end. The first axial end has a hollow piercing member with a tip having an opening mounted thereon and the second axial end has an engaging member for releasably engaging a syringe, the hollow piercing member, the central hub, and the engaging member being in fluid communication with one another. The protractible luer adaptor is longitudinally slidable within the sleeve between a retracted position where the hollow piercing member is substantially contained within the central portion of the housing and an advanced position where the tip of the hollow piercing member extends into the vial socket.

Accordingly, there is a need for an improved medical reconstitution system that includes the advantages of known systems like that described above, but is less susceptible to damage or breakage during use than known medical reconstitution systems.

### SUMMARY

In accordance with the present invention, there is provided a medical reconstitution system as defined in claim 1, a method of delivering a multi-constituent composition as defined in claim 13, and a finger member as defined in claim 17.

Further advantages are achieved by the embodiments indicated by the dependent claims.

A reading of the following detailed description together with the drawings will provide a more thorough understanding of the invention.

### BRIEF DESCRIPTION OF THE DRAWINGS

Figure 1 is a longitudinal cross sectional view of a prior art medical reconstitution device;
Figure 2 is another longitudinal cross section of the prior art medical reconstitution device of Figure 1 (shown as after activation of the piercing member);
Figure 3 is a perspective view of an injection syringe;
Figure 4 is a longitudinal cross sectional view of the syringe of Figure 3, including a partially removable sealing cap;
Figure 5 is a longitudinal cross sectional view of the syringe of Figure 4 with a removable portion of the sealing cap removed;
Figure 6 is a top perspective view of one embodiment of a distal finger member for a syringe;
Figure 7 is a bottom perspective view of the distal finger member of Figure 6;
Figure 8 is a plan view showing the top of the distal finger member shown in Figures 6 and 7;
Figure 9 is a cross sectional view of the distal finger member shown in Figures 6-8 taken along line 9-9 in Figure 8;
Figure 10 is a plan view showing the bottom of the distal finger member shown in Figures 6-9;
Figure 11 is a cross sectional view of the distal finger member shown in Figures 6-10 taken along line 11-11 in Figure 10;
Figure 12 is a longitudinal cross section of a medical reconstitution system according to the invention with its movable piercing member in a fully retracted position; and
Figure 13 is a longitudinal cross section of the medical reconstitution system of Figure 12 with the movable piercing member in a fully pierced position.

### DETAILED DESCRIPTION

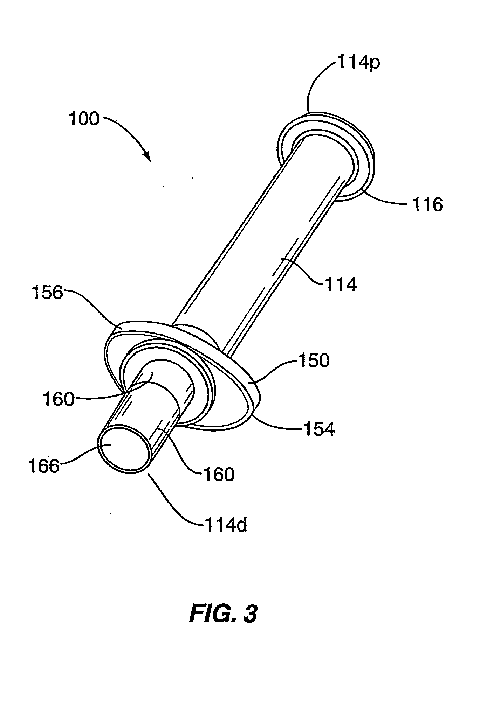

An injection syringe 100 is shown in Figures 3-5. As shown in Figure 3, the syringe includes a barrel 114, a proximal finger brace 116, and a distal end member or sealing cap 160 having a first connector 162. In the following description, the term "distal" is used to refer to a position or direction that is toward the injection end of the syringe 100, and the term "proximal" is used to refer to a position or direction that is distant from the injection or distal end of the syringe 100. The syringe 100 further includes a distal finger member or distal finger brace 150 that is positioned proximate to the distal end of the syringe 100. In other words, the distal finger member or brace 150 is positioned on the syringe barrel 114 such that the brace 150 is nearer the distal end 114d of the syringe barrel 114 than the opposed proximal end 114p of the barrel 114. The distal finger member 150 is configured to permit an axial force to be applied to the syringe 100 in a distal direction with one or more fingers of a person's hand or hands. As shown in Figure 3, the distal finger member 150 includes a first outwardly protruding tab portion 154, and an opposed second outwardly protruding tab portion 156.

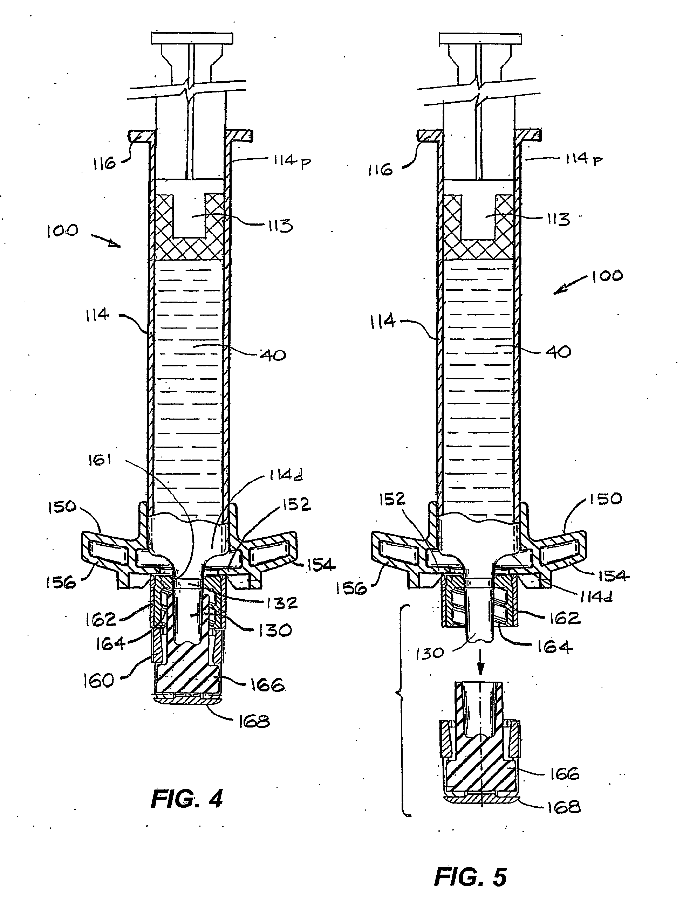

Referring to Figures 4 and 5, the syringe 100 includes an elongated barrel 114 having a plunger 113 disposed therein. The distal end of the barrel includes a tapered nipple 130 having a notch or groove 132 therearound. In a prefilled syringe 100, a flowable constituent 40 is disposed within the barrel 114. In order to seal and protect the flowable constituent 40 within the syringe 100, a tamper-evident sealing cap 160 may be secured on the syringe nipple 130 by the groove 132 as shown. One such sealing cap 160 that may be used with a syringe 100 is a compatible V-OVS® closure available from Vetter Pharma-Turm Inc., for example. As shown in Figures 4 and 5, the sealing cap 160 includes a retained cap-shaped portion 162 and a removable sealing portion 166. The retained cap-shaped portion 162 includes a collar portion 161 that is received in the groove 132, thereby positively retaining the cap-shaped portion 162 on the nipple 130 and barrel 114. The sealing portion 166 includes a resilient sealing member 166 that mates with and seals the open tip of the syringe nipple 130 when the sealing cap 160 is unopened, as shown in Figure 4. When the sealing portion 166 of the sealing cap 160 is removed from the retained portion 162 as shown in Figure 5, the syringe nipple 130 is unsealed, and capable of dispensing the flowable constituent 40 from the syringe 100.

As shown in Figure 5, the exposed nipple 130 and positively retained portion 162 of the sealing cap 160 combine to form a male luer-type connector as is known in the art. The tapered nipple 130 is sized and shaped to be received by a compatible female luer connector on a vial or other medical device. Threads 164 in the retained portion 162 of the cap 160 are configured to lockingly engage mating threads on a compatible female luer-type connector, thereby forming a leak-tight seal between the connectors.

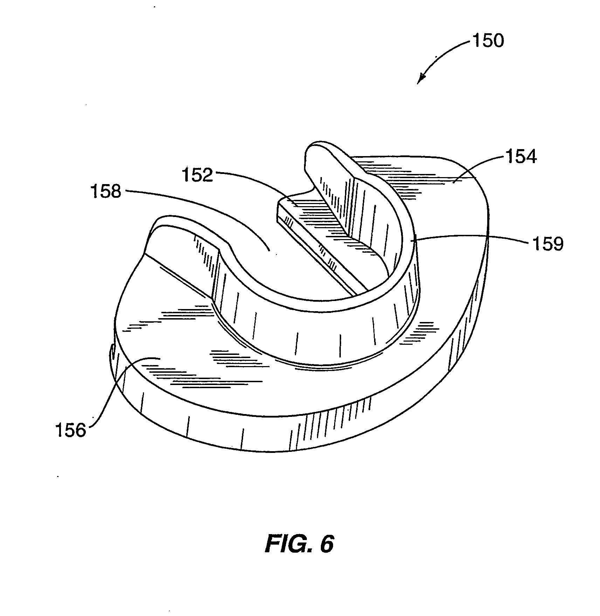

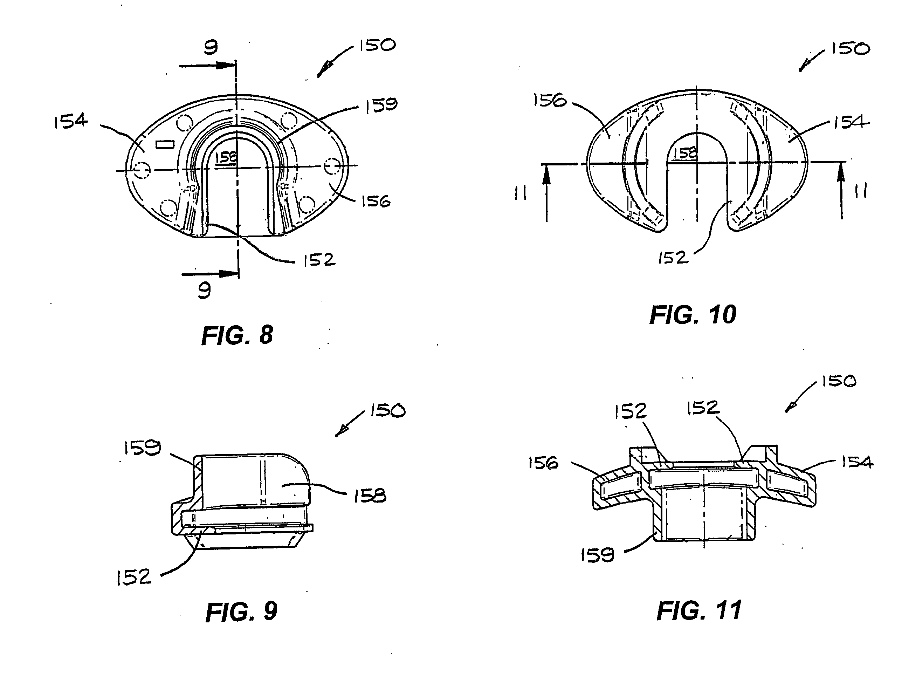

One embodiment of a distal finger member 150 for use with a syringe 100 is shown in Figures 4-11. In this embodiment, the distal finger member 150 is configured to securely snap onto the distal end of a pre-filled syringe 100 as shown in Figures 3-5. The distal finger member 150 includes a first tab portion 154 and an opposed second tab portion 156. The tab portions 154, 156 each are configured to receive at least one fingertip to apply a distally-directed force to the syringe 100. As shown in Figures 6-8 and 10, the distal finger member 150 includes a substantially U-shaped recess 158 including a central bore for receiving at least a portion of the distal end 114d of the syringe barrel 114 therein. A substantially cylindrical wall 159 at least partially surrounds a portion of the recess 158. As shown in Figures 6-9 and 11, the wall 159 preferably includes an inner bore having a diameter that is equal to or slightly less than the major diameter of a mating syringe barrel 114. When engaged on a syringe barrel 114, the cylindrical wall 159 grips the barrel to align and at least partially secure the distal finger member 150 on the barrel 114.

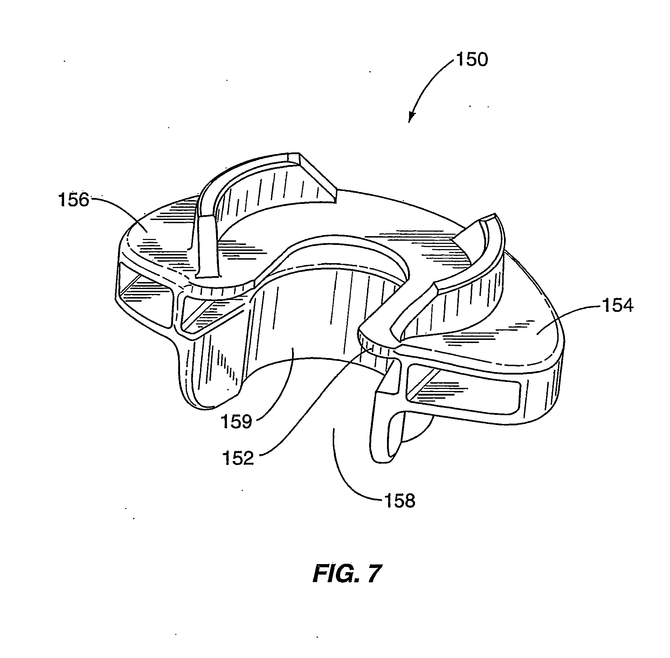

As shown in Figures 7-11, a substantially U-shaped lip 152 also surrounds the U-shaped recess 158 and is substantially coextensive therewith. The opposed edges of the U-shaped lip 152 are spaced apart by a distance that is smaller than the major diameter of a mating syringe barrel 114, and that is at least slightly larger than the largest diameter of the nipple 130 on the mating syringe barrel 114. As shown in Figures 4 and 5, the thickness of the lip 152 is selected such that the lip 152 is capable of being received in a space or gap between the distal end 114d of the syringe barrel 114 and the retained portion 162 of the sealing cap 160. The lip 152 functions to locate the distal finger member 150 on the distal end 114d of a syringe 100, to substantially prevent the finger member 150 from sliding in either a distal or proximal direction on the barrel 114, and to transfer a substantial distally-directed force from the distal finger member 150 to the syringe nipple 130 through the engaged connector 162. The positive engagement between the collar 161 of the connector 162 and the groove 132 in the nipple 130 prevents the connector 162 from disengaging from the nipple 130 as a substantial distally-directed force is applied to the distal finger member 150 and syringe 100.

Preferably, the distal finger member 150 is constructed of a resilient material that permits the U-shaped member 150 to elastically deform and recover as the member 150 is snapped onto the distal end 114d of a syringe barrel 114. For example, the distal finger member 150 may be formed of molded polypropylene or another suitable polymeric material.

The distal finger member 150 may have any suitable configuration that permits a substantial axial force to be applied to the distal end of a syringe in a distal direction at or near the distal end of the syringe with one or more fingers of a person's hand or hands. For example, the distal finger member 150 may include an outwardly protruding disc that substantially surrounds and positively engages the distal end of the syringe barrel, or any other suitable shape.

In an example of the syringe 100, the barrel 114 is a substantially transparent glass tube that permits a substantially unobstructed view of the syringe's contents 40. Alternatively, the barrel 114 may be constructed of a suitable polymeric material. The proximal finger brace 116 and plunger 113 may be constructed of any suitable material, such as a suitable polymeric material or the like, as is known in the art.

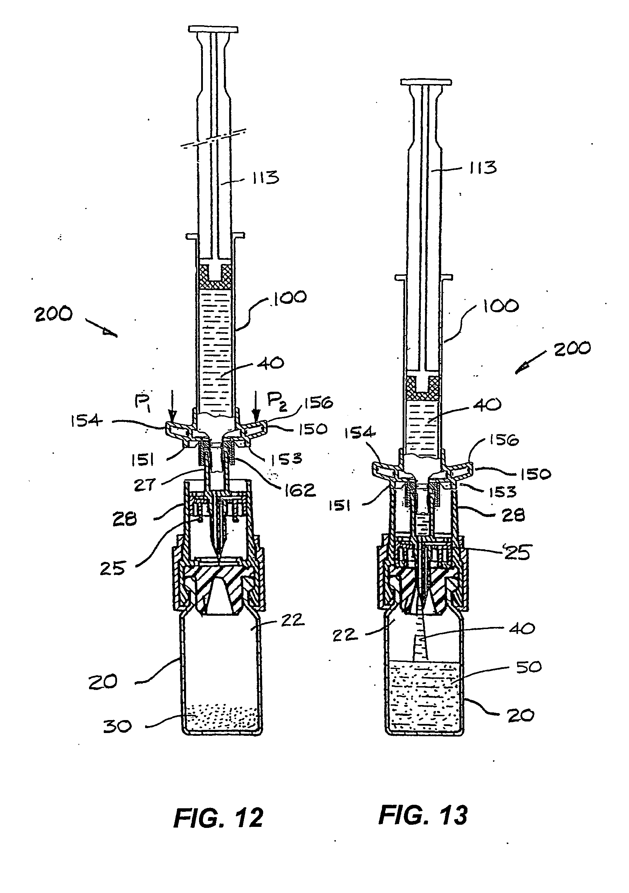

The syringe 100 described above is particularly well suited for use with a receptacle 20 having a movable piercing member 25 like that described above and shown in Figures 12 and 13. In order to reconstitute a powdered or freeze-dried first constituent 30 in a vial 22 with a flowable second constituent 40 in a syringe 100, the syringe 100 is aligned with the receptacle 20 and the first and second connectors 162, 27 are sealably engaged together. In practice, a person may support the receptacle 20 with one hand (such as on a flat, horizontal surface), while applying force or pressure to the distal finger member 150 in a distal direction with one or more fingers of the opposite hand. The distal direction of the force or pressure applied to the distal finger member 150 is indicated in Figure 12 by arrows P₁ and P₂.

As this distally-directed force or pressure is applied to the distal finger member 150 of the syringe 100, the movable piercing member 25 of the receptacle 20 is pushed downward until the movable piercing member 25 is in a fully-pierced position as shown in Figure 13. In this fully-pierced position, a fluid communication path is established between the barrel 114 of the syringe 100, and the vial 22 of the receptacle 20. In one embodiment of the system 200 as shown in Figure 13, at least a portion of the distal finger member 150 meets or contacts at least a portion of the receptacle 20 when the movable piercing member 25 reaches the fully-pierced position. For example, in the embodiment shown in Figure 13, the stops 151, 153 on the bottom of the distal finger member 150 of the syringe 100 contact the upper edge of the sterile shield 28 on the receptacle 20, thereby providing a positive indication to a user that the hidden movable piercing member 25 is in the fully-pierced, activated position.

Because the distally-directed force or pressure is applied to the distal finger member 150 at a location that is near the distal end of the syringe 100, even in instances where an eccentric or transverse load inadvertently is applied to the syringe in this distal location, any resultant bending moment on the engaged connectors 162, 27 or syringe barrel 114 is small compared to the bending moment that would result if the same transverse load was applied at or near the proximal end of the syringe 100. Accordingly, the improved syringe 100 and medical reconstitution system 200 provides a substantially lower risk of damage or breakage than known medical reconstitution devices and methods like the prior art device and method described above.

The syringe 100 and improved medical reconstitution system 200 described above is particularly important to frequent users of such systems, such as patients with hemophilia. Such persons must regularly inject themselves with an antihemophilic factor in order to improve blood coagulation. Such persons typically self-administer such injections at home. One such antihemophilic factor is recombinant KOGENATE® FS, produced by Bayer HealthCare LLC. Such a two-part recombinant hemophilic factor typically includes a dried active constituent and a compatible diluent. While prior art medical reconstitution systems like the system 10 described above provide a needleless system and method for reconstituting a recombinant antihemophilic factor such as KOGENATE® FS, the improved system 200 and method described above provides a system having all of the advantages of the prior system 10, plus the further advantage of a reduced likelihood of accidental damage or breakage of the syringe 100 or receptacle 20 during reconstitution. Certain embodiments of the improved system 200 can also provide a clear visual indication to a user when the system 200 has been fully activated, e.g. when stops 151 and 153 are in full or close contact with a component of a reconstitution system, e.g. such as sterile shield 28 (see Figure 13). Accordingly, the invention provides a reconstitution process that is simple, convenient, and safer than existing systems.

The various embodiments of the invention described above are provided for the purpose of illustrating various features and aspects of the invention, and are not intended to limit the scope of the invention. Persons of ordinary skill in the art will recognized that certain modifications may be made to the described embodiments without departing from the scope of the present invention. For example, a syringe 100 is described above as having a distal finger member 150 that is separate and removable from its mating syringe barrel 114, the finger member and syringe barrel may be integrally formed together of a suitable material. Alternatively, separate protruding finger tabs may be attached to an outer wall of the syringe barrel with a suitable adhesive or the like. All such modifications are intended to be within the scope of the appended claims.

## Claims

1. A medical reconstitution system (200) for a composition comprising first and second mixable constituents (30, 40), the system (200) comprising:
(a) a receptacle (20) containing the first constituent (30), the receptacle (20) comprising:
(i) a container (22) including a piercable seal (24);
(ii) a piercing member (25) configured to selectively pierce the piercable seal (24), the piercing member (25) being operable between a retracted position and a fully-pierced position; and
(iii) a first connector (27) connected to and in fluid communication with the piercing member (25);
(iv) a sterile shield (28) substantially surrounding the piercable seal (24) and piercing member (25);
(b) a syringe (100) containing the second constituent (40), the syringe (100) comprising:
(i) a barrel (114) having a distal end (114d), a proximal end (114p), and a second connector (162) on the distal end (114d), the second connector (162) being configured to engage the first connector (27) of the receptacle (20) and provide a substantially fluid-tight connection therewith;
**characterized in that**
(ii) at least one finger member (150) non-slidably attached to a distal portion of the barrel (114), the finger member (150) being configured to permit application of a force by one or more fingers of a person's hand such that the syringe (100) is urged in a distal direction;
(c) wherein when the piercing member (25) is in the retracted position and the first and second connectors (27, 162) are sealingly engaged together, the piercing member (25) is capable of being advanced to the fully-pierced position by pushing and displacing the finger member (150) in the distal direction toward the receptacle (20).

2. A system (200) according to claim 1 wherein the first constituent (30) comprises an antihemophilic factor and the second constituent (40) comprises a diluent therefor.

3. A system (200) according to claim 1 or 2, further comprising an injection needle (26) configured to sealingly connect to the second connector (162) of the syringe (100).

4. A system (200) according to claims 1-3 wherein at least a portion of the finger member (150) of the syringe (100) contacts a portion of the sterile shield (28) when the piercing member (25) is advanced to the fully pierced position.

5. A system (200) according to one of the claims 1 to 4 wherein the barrel (114) of the syringe (100) comprises glass.

6. A system (200) according to one of the claims 1 to 5 wherein the syringe (100) further comprises a plunger (113) disposed in the barrel (114).

7. A system (200) according to one of the claims 1 to 6 wherein the first and second connectors (27,162) are compatible luer-lock connectors.

8. A system (200) according to one of the claims 1 to 7 wherein the finger member (150) comprises:
(a) a first finger tab (154) on a first side of the barrel (114); and
(b) a second finger tab (156) on a second side of the barrel (114).

9. A system (200) according to claim 8 wherein the first and second sides of the barrel (114) are substantially opposite each other.

10. A system (200) according to one of the claims 1 to 9 wherein the finger member (150) comprises a polymeric material.

11. A system (200) according to claim 1, further comprising a pharmaceutical composition within the barrel (114).

12. A system (200) according to one of the claims 1 to 11 wherein the finger member (150) is removably attached to the distal portion of the barrel (114).

13. A method of delivering a multi-constituent composition for injection, the method comprising:
(a) providing a first constituent (30) in a receptacle (20) having a piercable seal (24), a piercing member (25) movable between a retracted position and a fully-pierced position, and a first connector (27) in fluid communication with the piercing member. (25);
(b) providing a second constituent (40) in a syringe (100) having a second connector (162) on the distal end (114d) of the syringe (100);
(c) connecting the first and second connectors (27, 162) while the piercing member (25) is in the retracted position;
(d) displacing the piercing member (25) from the retracted position to the fully-pierced position, thereby causing the piercing member (25) to pierce the piercable seal (24);
(e) injecting the second constituent (40) from the syringe (100) into the receptacle (20) through the first and second connectors (27, 162) and piercing member (25);
(f) causing the first and second constituents (30, 40) to mix together in the receptacle (20) to form the composition; and
(g) drawing the composition into the syringe (100) from the receptacle (20) through the piercing member (25) and through the first and second connectors (27, 162);
**characterized in that**
(h) the syringe (100) has at least one finger member (150) proximate to a distal end (114d) of the syringe (100); and
(i) the displacing of the piercing member (25) according to step (d) from the retracted position to the fully-pierced position is performed by pushing the finger member (150) toward the receptacle (20).

14. The method according to claim 13 wherein at least a portion of the finger member (150) of the syringe (100) meets a portion of the receptacle (20) when the piercing member (25) is in the fully-pierced position to provide a visual indication to a user that the syringe (100) is fully engaged with the receptacle (20).

15. The method according to claim 13 or 14 wherein the syringe (100) and receptacle (20) are substantially transparent such as to substantially permit viewing of the first and second constituents (30, 40) and mixtures thereof within the receptacle (20) and syringe (100).

16. The method according to one of the claims 13 to 15 wherein the first constituent (30) is an antihemophilic factor and the second constituent (40) is a compatible diluent therefor.

17. A finger member (150) for a syringe (100) having a barrel (114) with a proximal end (114p) and a distal end (114d), the finger member (150) comprising:
(a) a central bore sized and configured to receive at least a portion of the barrel (114) proximate to the distal end (114d) of the barrel (114);
(b) a lip (152) configured to positively engage the distal end (114d) of the barrel (114); and
(b) at least one outwardly extending finger tab (154, 156);
**characterized in that**
the finger member (150) further comprises a U-shaped recess (158) that permits the finger member (150) to be engaged onto the barrel (114) from a side of the barrel (114).

18. A finger member (150) according to claim 17, further comprising at least one upwardly extending wall (159) that at least partially surrounds the central bore.

19. A finger member (150) according to claim 17 or 18, further comprising at least one downwardly extending stop (151, 153).

20. A finger member (150) according to one of the claims 17 to 19, further comprising a resilient polymeric material.

## Patentansprüche

1. Medizinisches Rekonstitutionssystem (200) für eine Zusammensetzung, die einen ersten und zweiten mischbaren Bestandteil (30, 40) umfasst, wobei das System (200) Folgendes umfasst:
(a) ein Gefäß (20), das den ersten Bestandteil (30) enthält, wobei das Gefäß (20) Folgendes umfasst:
(i) einen Behälter (22), der ein durchstechbares Dichtungselement (24) enthält;
(ii) ein Durchstechelement (25), das ausgebildet ist, um das durchstechbare Dichtungselement (24) wahlweise zu durchstechen, wobei das Durchstechelement (25) zwischen einer herausgezogenen Position und einer vollständig durchgestochenen Position hin- und herbewegbar ist; und
(iii) ein erstes Verbindungselement (27), das mit dem Durchstechelement (25) verbunden und in Fluidkommunikation mit diesem ist;
(iv) eine sterile Abschirmung (28), die das durchstechbare Dichtungselement (24) und das Durchstechetement (25) im Wesentlichen umgibt;
(b) eine Spritze (100), die den zweiten Bestandteil (40) enthält, wobei die Spritze (100) Folgendes umfasst:
(i) einen Zylinder (114) mit einem distalen Ende (114d), einem proximalen Ende (114p) und einem zweiten Verbindungselement (162) auf dem distalen Ende (114d), wobei das zweite Verbindungselement (162) ausgebildet ist, um mit dem ersten Verbindungselement (27) des Gefäßes (20) in Eingriff zu gelangen und eine im Wesentlichen fluiddichte Verbindung mit diesem bereitzustellen;
**dadurch gekennzeichnet, dass**
(ii) zumindest ein Fingerelement (150), das nicht verschiebbar an einem distalen Abschnitt des Zylinders (114) angebracht ist, wobei das Fingerelement (150) ausgebildet ist, um die Ausübung einer Kraft mittels eines oder mehrerer Finger der Hand einer Person zu ermöglichen, sodass die Spritze (100) in eine distale Richtung gedrückt wird;
(c) worin, wenn sich das Durchstechelement (25) in zurückgezogener Position befindet und das erste und zweite Verbindungselement (27, 162) dichtend in Eingriff miteinander sind, das Durchstechelement (25) in der Lage ist, durch Drücken und Verschieben des Fingerelements (150) in die distale Richtung zum Gefäß (20) hin in die vollständig durchgestochene Position vorgeschoben zu werden.

2. System (200) nach Anspruch 1, worin der erste Bestandteil (30) einen antihämophilen Faktor umfasst und der zweite Bestandteil (40) einen Verdünner dafür umfasst.

3. System (200) nach Anspruch 1 oder 2, weiters umfassend eine Injektionsnadel (26), die ausgebildet ist, um mit dem zweiten Verbindungselement (162) der Spritze (100) auf abgedichtete Weise verbunden zu werden.

4. System (200) nach Anspruch 1 bis 3, worin zumindest ein Abschnitt des Fingerelements (150) der Spritze (100) einen Abschnitt der sterilen Abschirmung (28) kontaktiert, wenn das Durchstechelement (25) in seine vollständig durchgestochene Position vorgeschoben wird.

5. System (200) nach einem der Ansprüche 1 bis 4, worin der Zylinder (114) der Spritze (100) Glas umfasst.

6. System (200) nach einem der Ansprüche 1 bis 5, worin die Spritze (100) weiters einen in dem Zylinder (114) angeordneten Kolben (113) umfasst.

7. System (200) nach einem der Ansprüche 1 bis 6, worin das erste und das zweite Verbindungselement (27, 162) kompatible Luer-Lock-Verbindungselemente sind.

8. System (200) nach einem der Ansprüche 1 bis 7, worin das Fingerelement (150) Folgendes umfasst:
(a) eine erste Fingerauflagefläche (154) an einer ersten Seite des Zylinders (114); und
(b) eine zweite Fingerauflagefläche (156) an einer zweiten Seite des Zylinders (114).

9. System (200) nach Anspruch 8, worin die erste und die zweite Seite des Zylinders (114) im Wesentlichen einander gegenüberliegen.

10. System (200) nach einem der Ansprüche 1 bis 9, worin das Fingerelement (150) ein polymeres Material umfasst.

11. System (200) nach Anspruch 1, weiters umfassend eine pharmazeutische Zusammensetzung in dem Zylinder (114).

12. System (200) nach einem der Ansprüche 1 bis 11, worin das Fingerelement (150) abnehmbar an dem distalen Abschnitt des Zylinders (114) angebracht ist.

13. Verfahren zur Verabreichung einer mehrere Bestandteile enthaltenden Zusammensetzung zur Injektion, wobei das Verfahren Folgendes umfasst:
(a) die Bereitstellung eines ersten Bestandteils (30) in einem Gefäß (20) mit einem durchstechbaren Dichtungselement (24), ein Durchstechelement (25), das zwischen einer herausgezogenen Position und einer vollständig durchgestochenen Position bewegbar ist, und ein erstes Verbindungselement (27) in Fluidkommunikation mit dem Durchstechelement (25);
(b) die Bereitstellung eines zweiten Bestandteils (40) in einer Spritze (100) mit einem zweiten Verbindungselement (162) an dem distalen Ende (114d) der Spritze (100);
(c) das Verbinden des ersten und des zweiten Verbindungselements (27, 162) während sich das Durchstechelement (25) in zurückgezogener Position befindet;
(d) das Verschieben des Durchstechelements (25) von der zurückgezogenen Position in die vollständig durchgestochene Position, wodurch bewirkt wird, dass das Durchstechelement (25) das durchstechbare Dichtungselement (24) durchsticht;
(e) das Injizieren des zweiten Bestandteils (40) aus der Spritze (100) in das Gefäß (20) durch das erste und das zweite Verbindungselement (27, 162) und das Durchstechelement (25);
(f) Bewirken, dass der erste und der zweite Bestandteil (30, 40) in dem Gefäß (20) miteinander vermischt werden, um die Zusammensetzung zu bilden; und
(g) das Ziehen der Zusammensetzung in die Spritze (100) aus dem Gefäß (20) durch das Durchstechelement (25) und durch das erste und das zweite Verbindungselement (27, 162);
**dadurch gekennzeichnet, dass**
(h) die Spritze (100) zumindest ein Fingerelement (15) in der Nähe eines distalen Endes (114d) der Spritze (100) aufweist; und
(i) das Verschieben des Durchstechelements (25) gemäß Schritt (d) von der zurückgezogenen Position in die vollständig durchgestochene Position durch Drücken des Fingerelements (150) zum Gefäß (20) hin durchgeführt wird.

14. Verfahren nach Anspruch 13, worin zumindest ein Abschnitt des Fingerelements (150) der Spritze (100) auf einen Abschnitt des Gefäßes (20) trifft, wenn sich das Durchstechelement (25) in vollständig durchgestochener Position befindet, um einem Benutzer visuell anzuzeigen, dass sich die Spritze (100) mit dem Gefäß (20) in vollständigem Eingriff befindet.

15. Verfahren nach Anspruch 13 oder 14, worin die Spritze (100) und das Gefäß (20) im Wesentlichen transparent sind, um das Betrachten des ersten und zweiten Bestandteils (30, 40) und Gemische dieser in dem Gefäß (20) und der Spritze (100) im Wesentlichen zu erlauben.

16. Verfahren nach einem der Ansprüche 13 bis 15, worin der erste Bestandteil (30) ein antihämophiler Faktor ist und der zweite Bestandteil (40) ein kompatibler Verdünner davon ist.

17. Fingerelement (150) für eine Spritze (100) mit einem Zylinder (114) mit einem proximalen Ende (114p) und einem distalen Ende (114d), wobei das Fingerelement Folgendes umfasst:
(a) eine zentrale Bohrung, die so dimensioniert und ausgebildet ist, dass sie zumindest einen Abschnitt des Zylinders (114) in der Nähe des distalen Endes (114d) des Zylinders (114) aufnimmt;
(b) einen Rand (152), der gestaltet ist, um mit dem distalen Ende (114d) des Zylinders (114) positiv in Eingriff zu gelangen; und
(c) zumindest eine sich nach außen erstreckende Fingerauflagefläche (154, 156);
**dadurch gekennzeichnet, dass**
das Fingerelement (150) weiters eine U-förmige Vertiefung (158) umfasst, die den Eingriff des Fingerelements (150) mit dem Zylinder (114) von einer Seite des Zylinders (114) ermöglicht.

18. Fingerelement (150) nach Anspruch 17, weiters umfassend zumindest eine sich nach oben erstreckende Wand (159), die zumindest teilweise die zentrale Bohrung umgibt.

19. Fingerelement (150) nach Anspruch 17 oder 18, weiters umfassend zumindest ein sich nach unten erstreckendes Anschlagelement (151, 153).

20. Fingerelement (150) nach einem der Ansprüche 17 bis 19, weiters umfassend ein elastisches polymeres Material.

## Revendications

1. Système (200) de reconstitution médicale pour une composition comportant des premier et second constituants mélangeables (30, 40), le système (200) comportant :
(a) un réceptacle (20) contenant le premier constituant (30), le réceptacle (20) comportant :
(i) un récipient (22) comprenant un opercule perçable (24) ;
(ii) un élément de perçage (25) configuré pour percer sélectivement l'opercule perçable (24), l'élément de perçage (25) pouvant être actionné entre une position rétractée et une position de perçage complet ; et
(iii) un premier raccord (27) raccordé à l'élément de perçage (25) et en communication de fluide avec celui-ci ;
(iv) un écran stérile (28) entourant sensiblement l'opercule perçable (24) et l'élément de perçage (25) ;
(b) une seringue (100) contenant le second constituant (40), la seringue (100) comportant :
(i) un corps (114) ayant une extrémité distale (114d), une extrémité proximale (114p) et un second raccord (162) sur l'extrémité distale (114d), le second raccord (162) étant configuré pour engager le premier raccord (27) du réceptacle (20) et pour réaliser avec lui un raccordement sensiblement étanche aux fluides ;
**caractérisé en ce que**
(ii) au moins un élément (150) pour les doigts est relié de façon non coulissante à une partie distale du corps (114), l'élément (150) pour les doigts étant configuré pour permettre l'application d'une force par un ou plusieurs doigts de la main d'une personne afin que la seringue (100) soit sollicitée dans une direction distale ;
(c) dans lequel, lorsque l'élément de perçage (25) est dans la position rétractée et que les premier et second raccords (27, 162) sont engagés de façon étanche l'un avec l'autre, l'élément de perçage (25) peut être avancé jusque dans la position de perçage complet par poussée et déplacement de l'élément (150) pour les doigts dans la direction distale vers le réceptacle (20).

2. Système (200) selon la revendication 1, dans lequel le premier constituant (30) comprend un facteur anti-hémophile et le second constituant (40) comprend un diluant pour celui-ci.

3. Système (200) selon la revendication 1 ou 2, comportant en outre une aiguille d'injection (26) configurée pour se raccorder de façon étanche au second raccord (126) de la seringue (100).

4. Système (200) selon les revendications 1 à 3, dans lequel au moins une partie de l'élément (150) pour les doigts de la seringue (100) est en contact avec une partie de l'écran stérile (28) lorsque l'élément de perçage (25) est avancé jusque dans la position de perçage complet.

5. Système (200) selon l'une des revendications 1 à 4, dans lequel le corps (114) de la seringue (100) comprend du verre.

6. Système (200) selon l'une des revendications 1 à 5, dans lequel la seringue (100) comporte en outre un plongeur (113) disposé dans le corps (114).

7. Système (200) selon l'une des revendications 1 à 6, dans lequel les premier et second raccords (27, 162) sont des raccords à verrouillage Luer compatibles.

8. Système (200) selon l'une des revendications 1 à 7, dans lequel l'élément (150) pour les doigts comporte :
(a) une première patte (154) pour un doigt sur un premier côté du corps (114) ; et
(b) une seconde patte (156) pour un doigt sur un second côté du corps (114).

9. Système (200) selon la revendication 8, dans lequel les premier et second côtés du corps (114) sont sensiblement opposés l'un à l'autre.

10. Système (200) selon l'une des revendications 1 à 9, dans lequel l'élément (150) pour les doigts comprend une matière de type polymère.

11. Système (200) selon la revendication 1, comportant en outre une composition pharmaceutique à l'intérieur du corps (114).

12. Système (200) selon l'une des revendications 1 à 11, dans lequel l'élément (150) pour les doigts est relié de façon amovible à la partie distale du corps (114).

13. Procédé de distribution d'une composition à constituants multiples pour une injection, le procédé comprenant :
(a) la mise en place d'un premier constituant (30) dans un réceptacle (20) ayant un opercule perçable (24), un élément de perçage (25) mobile entre une position rétractée et une position de perçage complet, et un premier raccord (27) en communication de fluide avec l'élément de perçage (25) ;
(b) la mise en place d'un second constituant (40) dans une seringue (100) ayant un second raccord (162) sur l'extrémité distale (114d) de la seringue (100)
(c) le raccordement des premier et second raccords (27, 162) tandis que l'élément de perçage (25) est dans la position rétractée ;
(d) le déplacement de l'élément de perçage (25) de la position rétractée à la position de perçage complet, amenant ainsi l'élément de perçage (25) à percer l'opercule perçable (24) ;
(e) à injecter le second constituant (40) de la seringue (100) dans le réceptacle (20) à travers les premier et second raccords (27, 162) et l'élément de perçage (25) ;
(f) à provoquer le mélange entre des premier et second constituants (30, 40) dans le réceptacle (20) pour former la composition ; et
(g) à aspirer la composition depuis le réceptacle (20) jusque dans la seringue (100) à travers l'élément de perçage (25) et à travers les premier et second raccords (27, 162) ;
**caractérisé en ce que**
(h) la seringue (100) comporte au moins un élément (150) pour les doigts proches d'une extrémité distale (114d) de la seringue (100) ; et
(i) le déplacement de l'élément de perçage (25) conformément à l'étape (d) de la position rétractée jusqu'à la position de perçage complet est exécuté en poussant l'élément (150) pour les doigts vers le réceptacle (20).

14. Procédé selon la revendication 13, dans lequel au moins une partie de l'élément (150) pour les doigts de la seringue (100) rejoint une partie du réceptacle (20) lorsque l'élément de perçage (25) est dans la position de perçage complet afin de fournir à un utilisateur une indication visuelle du fait que la seringue (100) est en engagement complet avec le réceptacle (20).

15. Procédé selon la revendication 13 ou 14, dans lequel la seringue (100) et le réceptacle (20) sont sensiblement transparents afin de permettre sensiblement une observation des premier et second constituants (30, 40) et de leurs mélanges à l'intérieur du réceptacle (20) de la seringue (100).

16. Procédé selon l'une des revendications 13 à 15, dans lequel le premier constituant (30) est un facteur anti-hémophile et le second constituant (40) est un diluant compatible pour celui-ci.

17. Elément (150) pour les doigts pour une seringue (100) ayant un corps (114) pourvu d'une extrémité proximale (114p) et d'une extrémité distale (114d), l'élément (150) pour les doigts comportant :
(a) un alésage central dimensionné et configuré pour recevoir au moins une partie du corps (114) à proximité de l'extrémité distale (114d) du corps (114) ;
(b) une lèvre (152) configurée pour engager fermement l'extrémité distale (114d) du corps (114) ; et
(b) au moins une patte (154, 156) pour un doigt, s'étendant vers l'extérieur ;
**caractérisé en ce que**
l'élément (150) pour les doigts comporte en outre un évidement (158) en forme de U qui permet à l'élément (150) pour les doigts d'être engagé sur le corps (114) depuis un côté du corps (114).

18. Elément (150) pour les doigts selon la revendication 17, comportant en outre au moins une paroi (159) s'étendant vers le haut, qui entoure au moins partiellement l'alésage central.

19. Elément (150) pour les doigts selon la revendication 17 ou 18, comportant en outre au moins une butée (151, 153) s'étendant vers le bas.

20. Elément (150) pour les doigts selon l'une des revendications 17 à 19, comprenant en outre une matière élastique de polymère.
